# EUROPEAN PATENT SPECIFICATION

(11) **EP 4 051 347 B1**
(45) Date of publication and mention of the grant of the patent: **24.09.2025**
(21) Application number: 20800622.1
(22) Date of filing: 30.10.2020
(51) Int. Cl.: A61M 5/32, A61M 5/24

(54) **NEEDLE PROTECTION DEVICE COMPRISING SYRINGE CENTRING FEATURES AND DISASSEMBLY LOCK**
NADELSCHUTZVORRICHTUNG MIT SPRITZENZENTRIERMERKMALEN UND DEMONTAGESICHERUNG
DISPOSITIF DE PROTECTION D'AIGUILLE COMPRENANT DES ÉLÉMENTS DE CENTRAGE DE SERINGUE ET UN VERROU DE DÉMONTAGE

(30) Priority: 01.11.2019 GB 201915939; 01.11.2019 GB 201915938
(43) Date of publication of application: 07.09.2022
(60) Divisional application: 23150288.1
(73) Proprietor: Tech Group Europe Limited, Mulhuddart Dublin DK15009 (IE)
(72) Inventor: MCELROY, Terry, Dublin, DK15009 (IE); HEALY, Karl, Dublin, DK15009 (IE); DOWLING, Patrick, Dublin, DK15009 (IE)
(74) Representative: Finlayson, Scott Henry
(86) International application number: PCT/EP2020/080574
(87) International publication number: WO 2021/084099

(56) References cited:
- FR-A1- 2 832 932
- US-A1- 2005 165 353
- US-A1- 2007 270 759
- US-A1- 2008 021 409
- US-A1- 2014 039 406

## Description

### Technical Field

The present invention relates generally to a needle protection device for a syringe. The needle protection device comprises syringe centring features and/or a disassembly lock configured to prevent spontaneous or accidental disassembly of the device. The invention also relates to assembled injection devices comprising a pre-filled syringe and a needle protection device. Associated methods of centring a syringe in a safety device and/or preventing accidental disassembly of the device are also provided.

### Background

To prevent needle stick injuries after completion of an injection, safety devices that act to cover the needle of a syringe or injection device after use have been developed. Such safety devices may consist of an accessory into which a pre-filled syringe may be inserted, which is configured to either retract the syringe into the housing, such that an injection end of the needle is not exposed, or advance a sleeve over the needle, optionally locking the sleeve in the advanced position to prevent subsequent retraction of the sleeve and exposure of the needle after injection.

An example of a needle safety device that extends a sleeve over the needle after completion of an injection process is described in WO2012/166527. The needle safety device described in this document comprises a protector support into which a pre-filled syringe can be inserted, and a needle sleeve that is advanced by a spring over the needle of the syringe once an injection has been completed. Further examples of the prior art are disclosed in US2005/165353A1, US2008/021409A1, US2007/270759A1 and FR283232A1.

### Summary

In a first aspect there is provided a safety device for a syringe, the safety device comprising: a syringe housing having a main body comprising a generally cylindrical portion configured to receive a syringe and a flange support configured to support a flange of the syringe. The flange support is configured to prevent or limit movement of the syringe relative to the syringe housing once the syringe has been inserted into the housing. In at least some embodiments, said flange support comprising a first abutment surface to limit proximal movement of the syringe relative to the housing, and a second abutment surface, configured to limit distal movement of the syringe relative to the housing. The device further comprises a needle shield comprising a sleeve telescopically arranged with respect to the syringe housing, wherein an inner diameter of the needle shield is larger than an outer diameter of the housing such that the needle shield can slide over the syringe housing between a retracted position in which the needle tip is exposed and an extended position in which a needle tip of the syringe is covered by the needle shield. Preferably, the safety device further comprises a locking mechanism to lock the needle shield in the extended position after injection. The syringe housing further comprises a plurality of resiliently deformable arms, each biased towards a first position in which an inner contact surface of the arm is positioned radially inwards of an inner surface of the generally cylindrical portion. This arrangement is configured to form a constriction within the syringe housing between the arms. The arms are configured to be deflected radially outwardly from the first position by the syringe when the syringe is inserted into the syringe housing. The inner contact surfaces of the arms are located at an intermediate position between proximal and distal ends of the syringe housing. The intermediate position of the arms may allow the arms to center the syringe body.

In at least some embodiments, the constriction formed between the arms is positioned in a proximal half or a proximal third of the syringe housing, distal of the flange support.

In some embodiments, each of the arms can comprise a cantilever arm extending from a fixed end connected to the housing body to a free end. In one example, the fixed end of the cantilever arm is located distal of the free end of the arm. This configuration can be particularly advantageous in facilitating removal of a moulded syringe housing from a mould. In other embodiments, the fixed end of each arm may be located proximal of the free end.

Optionally, the generally cylindrical body can comprise a plurality of windows or cut-outs formed in a wall of the cylindrical body, and wherein each of the arms is positioned in a window or cut-out.

In some embodiments, the safety device can comprise two diametrically opposed arms. However, three or more arms may be provided.

Optionally, the cantilever arms can comprise a curved longitudinal cross-sectional profile in which each arm curves inwardly towards a longitudinal axis of the housing in a first portion, and curves outwardly away from the longitudinal axis in a second portion, and wherein the contact surface of each arm is located at an inflection point of the curve. In other words, the arms may comprise a substantially S-shaped portion.

The safety device is adapted for use with conventional manual syringes, or similar syringes. Accordingly, the safety device may further comprise a manually activated plunger.

Optionally, a biasing element may be provided, and configured to bias the needle shield into the extended position with respect to the syringe housing. The biasing element may be arranged between first and second opposing support surfaces provided on the syringe housing and the needle shield respectively.

Components of the safety device may be formed of a polymeric material and may advantageously be injection moulded. In at least some examples, the housing and the arms are formed as a single moulded part.

The arms may be configured to centre a syringe barrel within the housing by biasing the syringe barrel into a position in which a longitudinal axis of the syringe barrel is coincident with a longitudinal axis of the housing.

In some embodiments, a locking mechanism is provided, which is configured to lock the needle shield in the extended position. A further releasable locking mechanism may be provided to maintain the needle shield in the retracted position relative to the syringe housing until an injection has been completed and the user releases the locking mechanism.

By providing a plurality of resilient arms as described herein, aspects of the present disclosure may provide a safety device to be able to securely accommodate syringes for which an outer diameter of the syringe (or syringe assembly components) may vary, due to manufacturing tolerances or design choices

In a second aspect there is provided an injection apparatus comprising the safety device as described with reference to any of the embodiments above, and further comprising a syringe inserted into the syringe housing. In some embodiments, the syringe further comprises a needle cap covering a needle of the syringe. The needle cap may be a rigid needle shield. In at least some embodiments, the outer diameter of the needle cap is larger than or equal to an outer diameter of the syringe barrel.

In a third aspect there is provided a method for manufacturing or assembly of a safety device. The method comprises providing, optionally by moulding, a first part comprising: a main housing body comprising a generally cylindrical portion configured to at least partially surround the barrel of a syringe inserted into the housing; a flange support configured to support a flange of the syringe; and a plurality of resiliently deformable arms biased towards a first position in which an inner contact surface of the arms is positioned radially inwards of the inner surface of the generally cylindrical portion to form a constriction within the housing, wherein the arms are configured to be deflected radially outwardly from the first position by a syringe inserted into the housing, and wherein the inner contact surfaces of the arms are located at an intermediate position between proximal and distal ends of the housing. The method further comprises: providing, optionally by moulding, a second part comprising a needle shield comprising a sleeve having an inner diameter that is larger than an outer diameter of the housing. The method also includes assembling the needle shield on the housing such that the sleeve is telescopically arranged with respect to the housing such that the needle shield can slide over the housing between an extended position and a retracted position; arranging biasing means between the needle shield and the syringe housing to bias the needle shield into the extended position; and releasably securing the needle shield in the retracted position.

Optionally, each of the arms may comprise a cantilever arm extending from a fixed end connected to the housing body to a free end. In at least some embodiments, the method further comprises inserting a syringe into the housing; deflecting the arms outwardly from the first position into a second position during insertion of the syringe.

In some embodiments, the syringe may comprise a syringe barrel having a flange, a needle, and a needle cap covering the needle. The needle cap can comprise a rigid needle shield. Optionally, an outer diameter of the needle cap is larger than an outer diameter of the syringe barrel.

The method may further comprise deflecting, with the needle cap, the arms in a radially outward direction to a second position during insertion of the syringe into the housing, and wherein arms return to the first position or an intermediate position, between the first position and the second position, as the syringe is seated in a final position within the housing, with the flange confined between the first and second abutment surfaces.

In a fourth aspect there is provided a method of assembling a safety device and centering a syringe therein. According to the fourth aspect of the invention, the method comprises providing a syringe housing comprising a main housing body having a generally cylindrical portion configured to at least partially surround the barrel of a syringe inserted into the housing; a flange support configured to support a flange of the syringe; and a plurality of resiliently deformable arms biased towards a first position in which an inner contact surface of the arms is positioned radially inwards of the inner surface of the generally cylindrical portion to form a constriction within the housing, wherein the arms are configured to be deflected radially outwardly from the first position by a syringe inserted into the housing, and wherein the inner contact surfaces of the arms are located at an intermediate position between proximal and distal ends of the housing. Inserting a syringe into the syringe housing to deflect the cantilever arms radially outwardly; and securing the syringe within the syringe housing.

The syringe may be secured within the syringe housing by engaging a flange of the syringe with a flange support provided at a distal end of the syringe housing.

In accordance with the invention, there is provided: a safety device for a syringe, the safety device comprising: a syringe housing having a main body comprising a generally cylindrical portion configured to receive a syringe and a flange support configured to support a flange of the syringe inserted into the syringe housing and limit axial movement of the syringe relative to the housing. The safety device also comprises a needle shield comprising a sleeve telescopically arranged with respect to the syringe housing, wherein an inner diameter of the needle shield is larger than an outer diameter of the housing such that the needle shield can slide over the housing between a retracted position in which a needle tip of the syringe is exposed, and an extended position in which the needle tip of the syringe is covered. In a first embodiment, the main body includes a track comprising a cut-out or groove in the main body and the needle shield comprises a pin configured to be received in the track and to move along the track from a first end of the track to a second end of the track as the needle shield moves between the retracted and extended positions. In other embodiments, the location of the pin and the track may be switched. For example, the pin may be provided on the syringe housing and the track may be provided on the syringe housing.

The main body further comprises a locking recess comprising a proximal latching surface and a distal latching surface. The needle shield further comprises a latch arm configured to engage the recess and having a proximal stop surface and a distal stop surface.

The device is configured such that when the needle shield is in an extended position, the latch arm engages the locking recess such that proximal movement of the needle shield relative to the syringe housing is limited (or prevented) by abutment of the proximal stop surface and the proximal latching surface, and distal movement of the needle shield relative to the syringe housing is limited or prevented by abutment of the distal stop surface and the distal latching surface. It will be appreciated that the distance between the proximal and distal stop surfaces may be substantially equal to the distance between the proximal and distal edge surfaces of the recess such that the latch arm fits tightly within the recess and axial movement of the syringe housing relative to the needle shield is prevented. In other embodiments, the distance between the proximal and distal edge surfaces may be larger than the distance between the proximal and distal latch surfaces such that movement of the syringe housing relative to the needle shield is permitted (e.g. to a small degree) but limited, for example, such that the needle of a syringe mounted in the syringe housing cannot be re-exposed after use.

By providing proximal and distal latching surfaces and proximal and distal stops on a latch arm, embodiments of the present invention may prevent accidental or spontaneous disassembly of the device by distal movement of the needle shield with respect to the housing.

In some embodiments, the distal stop surface can be angled with respect to a central longitudinal axis of the syringe housing, wherein an obtuse angle is formed between the distal stop surface and the longitudinal axis.

The distal latching surface may be angled with respect to a central longitudinal axis of the syringe housing, and wherein an acute angle is formed between the distal latching surface and the longitudinal axis.

An angle between the distal latch surface and an axis perpendicular to the longitudinal axis may be between 10 and 20 degrees, for example approximately 15 degrees. An angle between the distal stop surface and an axis perpendicular to the longitudinal axis may be between 10 and 20 degrees, for example approximately 15 degrees.

The angle of the distal latch surface and the distal stop surface may be corresponding. That is, the distal latch surface and the distal stop surface may extend in parallel planes.

Additionally or alternatively, the proximal stop surface may be angled with respect to a longitudinal axis of the syringe housing, wherein an obtuse angle is formed between the proximal stop surface and a central longitudinal axis of the syringe housing.

The proximal latching surface may be angled with respect to a longitudinal axis of the syringe housing, wherein an acute angle is formed between the proximal latching surface and the longitudinal axis.

An angle between the proximal latch surface and an axis perpendicular to the longitudinal axis may be between 10 and 20 degrees, for example approximately 15 degrees. An angle between the proximal stop surface and an axis perpendicular to the longitudinal axis may be between 10 and 20 degrees, for example approximately 15 degrees.

The angle of the proximal latch surface and the proximal stop surface may be corresponding. That is, the proximal latch surface and the proximal stop surface may extend in parallel planes.

In some embodiments, the pin may comprise an undercut, e.g. between the pin and a wall of the needle shield. Optionally, the undercut can be formed by providing a distal surface of the pin that is angled with respect to the longitudinal axis, wherein an obtuse angle is formed between the distal surface of the pin and the longitudinal axis.

Additionally or alternatively, the track can comprise a distal end wall, said distal end wall facing in a proximal direction, and wherein the distal end wall is angled with respect to the longitudinal axis, and wherein an acute angle is formed between the distal end wall and the longitudinal axis.

In some embodiments, the needle shield can comprise a plurality of latch arms, each configured to engage a corresponding locking recess. For example, the plurality of latch arms may comprise two diametrically opposed latch arms.

Optionally, each latch arm is configured to engage the corresponding locking recess when the pin is located at the second end of the track.

In at least some embodiments, each latch arm can be resiliently deformable, and wherein the latch arm is initially configured to engage a proximal recess in the housing and is configured to be deflected by the housing (optionally a ramped surface on the housing) as the needle shield moves from the retracted to the extended position.

The safety device is adapted for use with conventional manual syringes, or similar syringes. Accordingly, the safety device may further comprise a manually activated plunger.

Optionally, the safety device may comprise a biasing element configured to bias the needle shield into the extended position.

Advantageously, the safety device described above may further comprise a plurality of deformable cantilever arms configured to centre a syringe body within the syringe housing. The cantilever arms may take the form of the cantilever arms described above with reference to the first aspect of the invention.

In a sixth aspect of the invention, the safety device may form part of an assembled injection apparatus. According to a sixth embodiment of the invention, there is provided an injection apparatus comprising any of the needle safety device embodiments described above, and further comprising a syringe inserted into the syringe housing.

In some embodiments, the syringe further comprises a needle cap covering a needle of the syringe. The needle cap may be a rigid needle shield. In at least some embodiments, the outer diameter of the needle cap is larger than or equal to an outer diameter of the syringe barrel.

Also in accordance with the invention, there is provided a method of securing an injection safety device against disassembly, wherein the method comprises: advancing a needle shield in a distal direction with respect to a syringe housing to an extended position in which the needle shield covers a needle of a syringe mounted in the syringe housing. The method further comprises: moving, by advancement of the needle shield to the extended position, a flexible latch arm provided on the needle shield into engagement with a locking recess in the syringe housing, the flexible latch arm comprising a proximal stop surface and a distal stop surface, the locking recess comprising a proximal latching surface and a distal latching surface. The method also includes: moving, by advancement of the needle shield to the extended position, a pin provided on the needle shield along a track provided on the syringe housing to bring the pin to a distal end of the track; limiting proximal movement of the needle shield relative to the syringe housing by abutment of the proximal stop surface and the proximal latching surface; and limiting distal movement of the needle shield relative to the syringe housing by abutment of the distal stop surface and the distal latching surface when the needle shield is in the extended position. Optionally, the method may further comprise the step of engaging the pin with an end wall of the track, for example by engaging an undercut formed in the pin with an end wall of the track.

In an eighth aspect there is provided: a method of manufacturing a safety device for a syringe, the method comprising: moulding a first part comprising: a syringe housing having a main body configured to receive a syringe, and a flange support configured to support a flange of the syringe to limit axial movement of syringe relative to the housing; forming, in the main body, a track configured to receive a pin and a locking recess comprising a proximal latching surface and a distal latching surface; moulding a second part comprising a needle shield comprising a sleeve having an inner diameter larger than an outer diameter of the main body, and forming, in the second part, a pin configured to be received in the track and a latch arm comprising a proximal stop surface and a distal stop surface; biasing the latch arm towards an extended position in which the latch arm moves into engagement with the locking recess, such that proximal movement of the needle shield relative to the syringe housing is limited by abutment of the proximal stop surface and the proximal latching surface and distal movement of the needle shield relative to the syringe housing is limited by abutment of the distal stop surface and the distal latching surface; assembling the needle shield onto the syringe housing such that the needle shield is telescopically arranged with respect to the housing and can slide over the syringe housing between a retracted position and the extended position, and wherein the pin is seated within the track; arranging a biasing element between the syringe housing and the needle shield to bias the needle shield into the extended position; and compressing the biasing element and securing the needle shield in a retracted position.

In a ninth aspect there is provided: a safety device for a syringe, the safety device comprising: a syringe housing having a main body comprising a generally cylindrical portion configured to receive a syringe and a flange support configured to support a flange of the syringe inserted into the syringe housing and limit axial movement of the syringe relative to the housing. The safety device also comprises a needle shield comprising a sleeve telescopically arranged with respect to the syringe housing, wherein an inner diameter of the needle shield is larger than an outer diameter of the housing such that the needle shield can slide over the housing between a retracted position in which a needle tip of the syringe is exposed, and an extended position in which the needle tip of the syringe is covered. In a first embodiment, the main body includes a track comprising a cut-out or groove in the main body and the needle shield comprises a pin configured to be received in the track and to move along the track from a first end of the track to a second end of the track as the needle shield moves between the retracted and extended positions. In other embodiments, the location of the pin and the track may be switched. For example, the pin may be provided on the syringe housing and the track may be provided on the syringe housing.

The main body further comprises a locking recess comprising a proximal latching surface and the needle shield further comprises a latch arm configured to engage the recess and having a proximal stop surface configured to engage the proximal latching surface. The pin further comprises an undercut configured to allow it to engage (e.g. in a hook relationship) a distal end wall of the track when the needle shield is in the extended position. By providing an undercut on the pin, the likelihood of the pin becoming disengaged from the track is reduced This may be particularly advantageous because disengagement of the pin from the track may allow disassembly of the device and exposure of a used needle.

Other advantages that may be provided by at least some embodiments of the present invention will also be apparent to the skilled person in light of the following detailed description and accompanying drawings.

### Brief description of the drawings

The following detailed description of the disclosure will be better understood when read in conjunction with the following drawings. It should be understood that the following drawings are nonlimiting, illustrative examples of ways in which the invention may be put into effect. However, the invention is defined by the appended claims and not limited to the exemplary embodiments shown in the drawings. In the drawings:
Fig. 1 shows an exploded view of a needle protection device and a syringe;
Fig. 2 shows a housing configured to receive a syringe;
Fig. 3A shows a longitudinal cross-sectional view of a proximal end of the housing of Figure 2 with a syringe barrel centred therein;
Fig. 3B shows an enlarged cross-sectional view of a cantilever arm from the housing shown in Fig. 3A;
Fig. 4A shows a transverse cross-sectional view of a centring arrangement according to a first embodiment;
Fig. 4B shows a transverse cross-sectional view of a syringe centring arrangement according to a second embodiment.
Fig. 4C shows a top perspective view of the distal end of the housing of Figure 2 according to one configuration that comprises optional additional rails at the distal end of the housing;
Fig. 5 shows a longitudinal cross-sectional view of the needle shield of the device shown in Fig. 1;
Figs. 6A-6C show the position of the needle shield with respect to a syringe housing of the safety device during use;
Fig. 7A shows a longitudinal cross-sectional view of the engagement between the needle shield and the syringe housing with the needle shield in the extended position, with a latch arm on the needle shield engaged with a locking recess in the housing;
Fig. 7B shows an enlarged view of the latch arms in fig. 7A;
Fig. 8 shows a further longitudinal cross-sectional view of the engagement between the needle shield and the housing with the needle shield extended to the advanced position, with a pin on the needle shield engaged with a track on the housing;
Fig. 9 shows, schematically, the steps of a method for manufacturing a safety device according to one aspect of the disclosure;
Fig. 10 shows, schematically, the steps of a method for manufacturing a safety device according to one aspect of the disclosure; and
Fig. 11 shows, schematically, the steps of a method for securing a safety device against disassembly according to one aspect of the disclosure.

### Detailed description

It will be understood that certain terminology is used in the following detailed description for convenience and is not limiting. The terms 'upper', 'lower', 'inner', 'outer', 'top' and 'bottom' may be used to designate directions in the drawings and relative positions of components parts or surfaces. Similarly, the terms 'inwardly', 'outwardly', 'upstream' and 'downstream' may be used to refer to directions that apply when the systems and methods are in use. The terms 'a', 'an' and 'the' should be read as meaning 'at least one'. The 'distal' direction herein is towards the needle of a conventional system mounted in the device. The 'proximal' direction is away from the needle of a conventional syringe, towards the flanged end of a conventional syringe mounted in the device.

The term 'comprising' will be understood to mean 'including but not limited to' such that systems or method comprising a particular feature or step are not limited to only those features or steps listed, but may also comprise features or steps not listed.

It will also be appreciated by those skilled in the art that modifications may be made to the exemplary embodiments described herein without departing from the invention. Structural features of systems and apparatuses described herein may be replaced with functionally equivalent parts. Moreover, it will be appreciated that features from the embodiments may be combined with each other without departing from the disclosure.

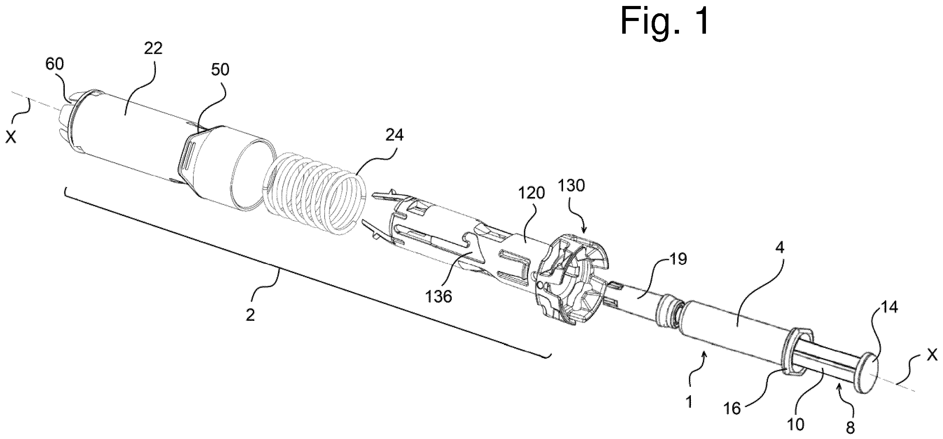

Turning now to Fig. 1, a safety device 2 for a syringe 1 generally comprises a syringe housing 120 configured to receive the syringe 1, and a needle shield 22 slidably mounted with respect to the housing 120, and configured to be advanced relative to the housing 120 to shield a needle of the syringe 1. The syringe comprises a syringe body 4, and a needle (not shown) which is fixed to a distal end of the body 4. A piston 8 is movably arranged with respect to the body 4 and may be advanced in a proximal direction to deliver a dose of medicament through the needle in the conventional manner. The syringe 1 may be a glass syringe of standard type, which is intended for a single use. The syringe 1 may comprise a staked syringe. However, the invention is not limited to glass and or staked syringes. As shown in Fig. 1, the syringe 1 extends from a distal end (needle end) to a proximal end.

The syringe body 4 preferably includes a flange 16 at the proximal end thereof. The flange 16 may be circumferential or may comprise two or more opposing projections that provide a surface against which the fingers may bear during manipulation of the syringe and injection of the medicament contained therein. The piston 8 generally comprises a rod 10 and push-surface 14 at the proximal end thereof against which the user can press a thumb to advance the piston 8 to deliver the injection. The needle of the syringe is advantageously covered before injection by a needle cap 19. The needle cap 19 may be a rigid needle shield (RNS). The rigid needle shield may be of the type described in United States Patent Application 13/813/590. However, other needle caps are possible.

As shown in Fig. 1, the safety device 2 extends along a longitudinal axis X-X and comprises the syringe housing 120, the needle shield 22 and a biasing element 24 arranged between the housing 120 and the needle shield 22 to bias the needle shield 22 into an extended position in which it extends beyond the distal tip of a syringe needle inserted into the device. When assembled for use, the safety device 2 comprises a syringe 1 inserted into the syringe housing 120. The syringe housing 120 is inserted into the needle shield 22.

The needle shield 22 comprises a generally tubular body having an inner diameter larger than an outer diameter of the syringe housing 120, such that the syringe housing 120 can be arranged within the needle shield 22. The needle shield 22 comprises an opening 60 at its distal end through which the needle of a syringe can extend. The needle shield 22 may optionally comprise a flange 50 at is proximal end. The flange 50 extends radially outwardly from the main body of the needle shield 22 and can comprise a bearing surface against which the user's fingers can rest during manipulation of the device, similar to the manner in which a user's fingers rest against the flange 16 of a conventional syringe.

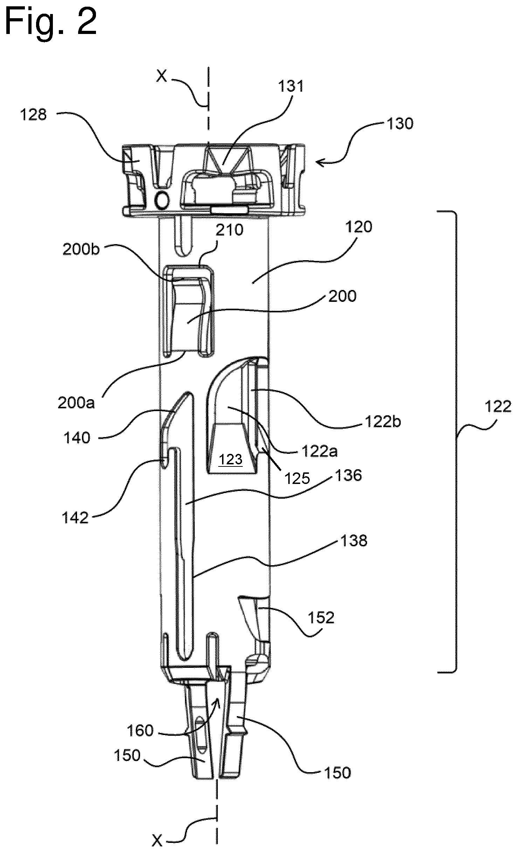

The syringe housing 120 comprises a main body having a generally cylindrical portion configured to at least partially surround the syringe body 4. The syringe housing 120 comprises an opening at its distal end through which a needle of the syringe 1 can extend, and a flange support 130 configured to support a flange 16 of the syringe 1. The flange support 130 is configured to prevent or limit axial movement of the syringe 1 relative to the syringe housing 120. One exemplary embodiment of a flange support 130 will be described in more detail with reference to Fig. 2. The syringe housing 120 can optionally comprise a track 136 along which a pin (not shown in Figure 1) on an interior surface of the needle shield 22 can slide. The track 136 is configured to define a path along which the pin of the needle shield can travel, thereby confining relative movement between the needle shield 22 and the syringe housing 120 to a predetermined path. The interaction between the track 136 and the pin (not shown) will be described in more detail with reference to Fig. 2.

The syringe housing 120 is arranged telescopically within the needle shield 22 and the biasing element 24 is arranged between these two parts. In the embodiment shown in Fig. 1, the biasing element 24 takes the form of a helical spring coiled around the syringe housing 120. The spring 24 can be arranged between a first support surface provided on the syringe housing 120 and a second support surface provided on the needle shield 22. Although the illustrated embodiments show a helical spring 24 arranged around the syringe housing 120, it will be appreciated that other biasing elements can be arranged to urge the needle shield 22 from its initially retracted position to its extended position.

A releasable locking mechanism is provided, which is configured to lock the position of the needle shield 22 with respect to the syringe housing 120 with the spring 24 compressed between the first and second support surfaces. Upon release of the releasable locking mechanism, the spring 24 is configured to urge the syringe housing 120 in a proximal direction with respect to the needle shield 22, such that the needle shield 22 is advanced over the exposed needle.

The syringe housing 120 will now be described in more detail with reference to Fig. 2. As shown in Fig. 2, the syringe housing 120 extends from the flange support 130 at the proximal end to an opening 160 at the distal end through which the needle extends. The syringe housing 120 comprises a main body 122 which takes the form of a hollow, generally cylindrical body configured to receive a syringe 1. As shown in Fig. 2, the main body 122 does not need to completely enclose the syringe body 4, as cut-outs and recesses may be provided, as will be described in more detail below.

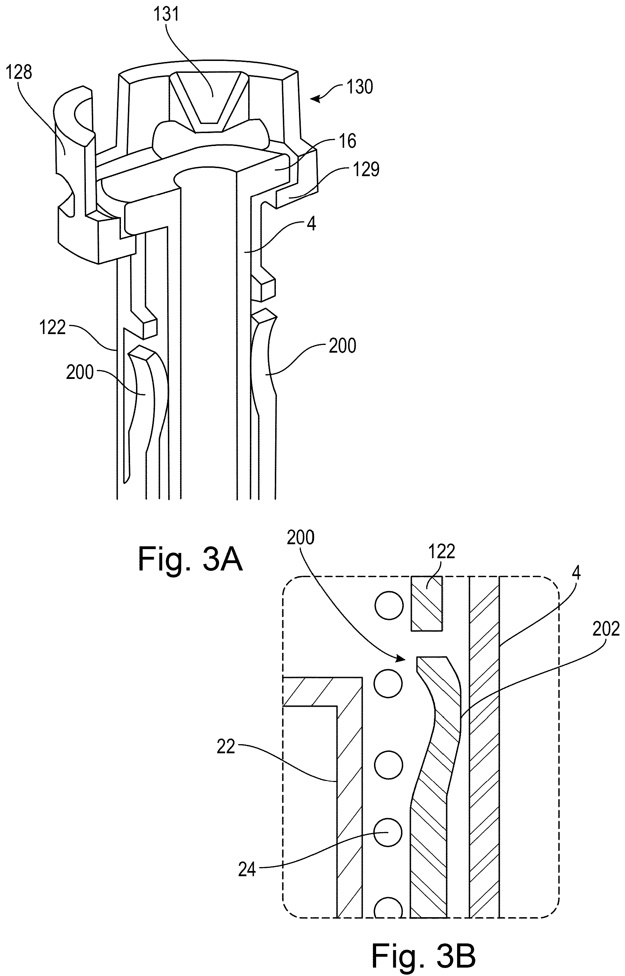

The main body 122 of the syringe housing 120 comprises a plurality of resiliently deformable arms 200. Referring now to Figs. 3A and 3B, each of the arms 200 comprises an inner surface 202 which faces radially inwards towards the longitudinal axis X-X of the device. The arms 200 are biased into a position in which an inner contact surface of the arms 200 is positioned radially inwards of the inner surface of the generally cylindrical body in such a way that a constriction is formed by the arms 200 in the housing 120. In other words, a closest distance between inner contact surfaces of the arms 200 is less than the inner diameter of the main body 122 of the housing 120.

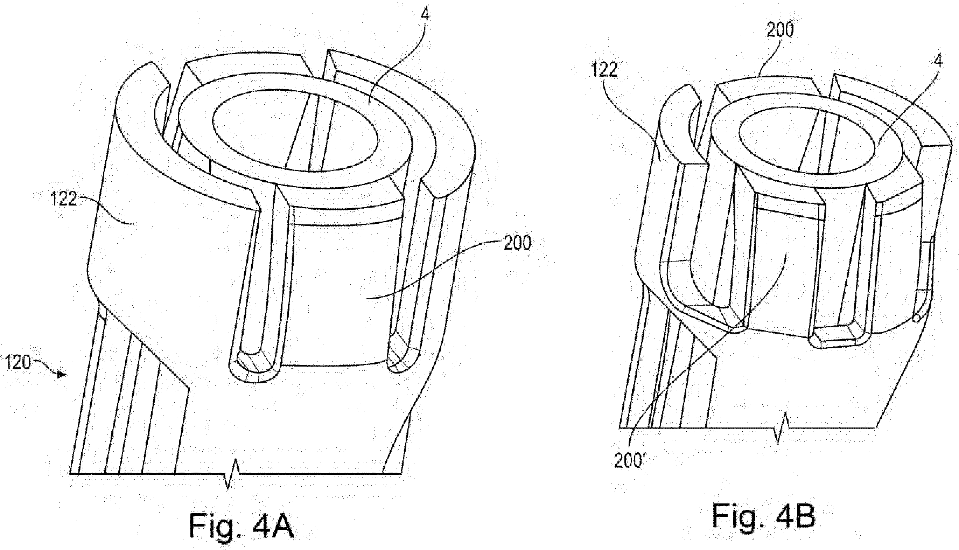

The arms 200 are formed of a resiliently deformably material to allow them to be deflected radially outwardly from the position to which they are biased and are configured to centre a syringe 1 in the device. This is particularly useful in cases where the syringe body 4 has an outer diameter that is significantly less than the inner diameter of the syringe housing 120. In such cases, although the syringe would not be secured against transverse movement (with respect to the longitudinal axis) by virtue of a snug fit within the housing 120, the restricted diameter between the arms 200 can be sufficiently small to contact the syringe body 4. Therefore, the arms 200 can act to centre the syringe 1 within the housing 120 (see Figs. 4A and 4B).

Although the restriction provided by the arms 200 may support syringes that would otherwise rattle in the housing 120, because the arms 200 can be deflected from their initial position, the syringe housing 120 is able to accommodate syringes within the syringe housing 120 that have an outer diameter that is smaller than the inner diameter of the syringe housing 120, but larger than the constriction formed by the arms 200. Upon insertion of a syringe having an outer diameter approaching the inner diameter of the housing 120, the arms 200 can deflect outwardly to accommodate the larger syringe. In such cases, the arms 200 improve the centring of the syringe and further reduce the extent to which the syringe may move in a transverse direction within the housing 120. Such an arrangement therefore provides for an improved syringe housing, adapted to centre and support a syringe within the housing, for a range of syringe diameters. The ability of the arms 200 to flex outwardly from their initial position may also be advantageous to allow a rigid needle shield having a diameter greater than the syringe body 4 to pass through the housing, whilst ensuring that the syringe body 4 is centrally seated once the syringe is fully inserted into the housing 120.

As shown in Fig. 2, the arms 200 can be formed integrally with the syringe housing 120. In the embodiment shown in Fig. 2, the arms 200 are each formed in a cut out 210 in the housing 120 and extend from a fixed end 200a attached to the housing 120 to a free end 200b disposed within the cut out 210. The arms 200 can be oriented with the free end 200b in the proximal direction and the fixed end 220a in the distal direction. This arrangement may be particularly advantageous in embodiments of the invention in which the housing 120 is formed by injection moulding parts. This is because the orientation of the arms 200 does not hinder (or hinders to a lesser extent) removal of the housing 120 from a mould in the proximal direction. Removal of the housing 120 from a mould in the proximal direction may be necessitated by other components of the housing 120, e.g. the flange support 130.

However, the skilled person will appreciate that the arms 200 may be provided in alternative configurations, whilst still providing the centring functionality described above. For example, the arms 200 may be oriented with the free end 200b in the distal direction, with the fixed end 200a in the proximal direction. It will also be appreciated that although the arms 200 illustrated in the drawings are configured as cantilever arms, extending from a fixed end to a free end, in some embodiments the arms may be fixed at both ends.

Referring again to Fig. 3A and 3B, the arms 200 may be curved along their length. For example, the arms 200 may comprise a curved longitudinal cross-sectional profile in which each arm 200 curves inwardly towards the longitudinal axis X-X of the housing in a first portion, and curves outwardly away from the longitudinal axis X-X in a second portion, and wherein the contact surface 202 of each arm 200 is located an inflection point of the curve. By providing cantilever arms curved in this manner, the syringe (and/or the rigid needle shield) can be guided through the constriction formed by the arms 200. The flared opening of the restriction that is formed by this curvature may prevent, e.g., a rigid needle shield from becoming caught on the arms 200 during insertion of the syringe 1 into the housing 120. For this reason, the flared opening tapers from its widest part towards the narrowest point of the constriction in a distal direction. It will be appreciated that although the arms 200 shown in the illustrated drawings comprise a smooth curved profile (which may be advantageous for manufacturing purposes), a flared opening to the restriction formed by the arms 200 may be formed by arms 200 having other geometrical profiles, e.g. an inflection point formed with intersecting straight portions.

As shown in Fig. 3B, the arms 200 can be configured such that they do not extend beyond the outer diameter of the main body 122 of the housing 120. This means that the arms 200 do not interrupt the interaction of the housing 120 with the needle shield 22, or with the biasing element 24.

Referring now to Fig. 4A and 4B, the plurality of arms may comprise two arms 200 diametrically opposed to each other (Fig. 4A) or three or more arms 200, configured to centre the syringe body 4 within the housing 120 (Fig. 4B). In the embodiment shown in Fig. 4B, a first arm 200 is provided on a first side of the housing, and two opposing arms 200' are arranged on an opposing side of the housing 120. As will be appreciated by the skilled person, in embodiments comprising two arms 200 (Fig. 4A) the arms 200 contact the syringe body 4 from opposing directions, restricting movement of the syringe body 4 back and forth in the direction defined between the two arms. However, in embodiments comprising three or more arms 200, movement of the syringe body 4 is restricted in further directions.

Referring again to Figs. 4A and 4B, the arms 200 can be formed with a curved transverse cross-section, to provide a convex inner surface 202, configured to conform to the cylindrical outer surface of the syringe body 4. The convex inner surface of the arms 200 can further limit the movement of the syringe body 4 away from alignment with the longitudinal axis X-X.

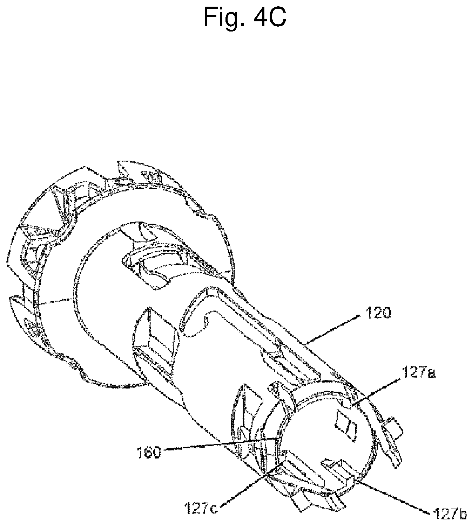

Referring to Figure 4C, to further facilitate centring of the syringe 1 within the syringe housing 120, the distal end of the syringe housing 120 may be provided with a plurality of longitudinally extending rails 127a, 127b, 127c, preferably at least three rails 127a, 127b, 127c. The rails 127a, 127b, 127c may extend from, or at least proximate to, the opening 160 at the distal end of the syringe housing 120 and extend longitudinally towards the proximal end of the syringe housing 120. The height, width, and length of the rails 127a, 127b, 127c are selected to ensure that the distal end of the syringe body 4 remains centred, such that the longitudinal axis of the syringe 1 is substantially coaxial with the longitudinal axis of the syringe housing 120 without inhibiting insertion of the distal end of the syringe body 4 through the opening 160 (i.e. an excessive force is not required to overcome the friction between the surface of the rails 127a, 127b, 127c and the outer surface of the distal end of the syringe body 4 when inserting the syringe 1 into the syringe housing 120). In a preferred embodiment, the rails 127a, 127b, 127c are located equidistantly about the inner circumference of the opening 160.

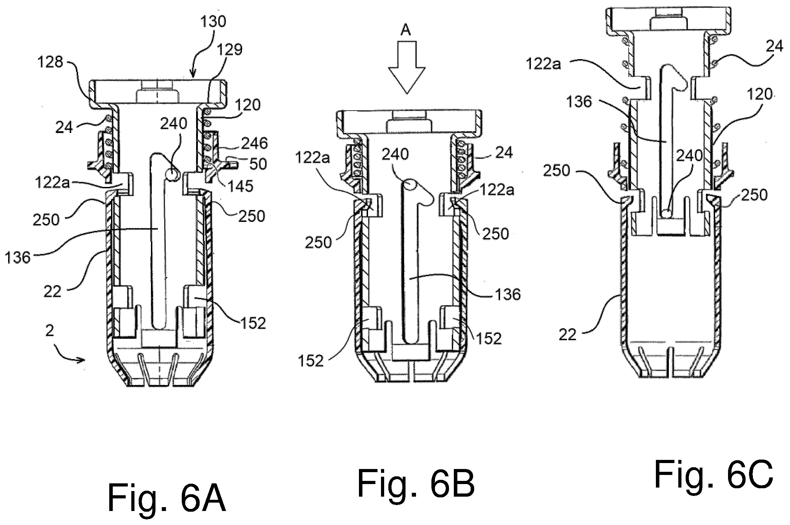

Returning again to Fig. 2, the syringe housing 120 comprises a flange support 130 configured to secure the syringe 1 in the housing 120 by confining the flange 16 of the syringe 1 between opposing abutment surfaces. As shown in Fig. 2, the flange support 30 comprises a circumferential wall 128 configured to surround the flange 16 of the syringe. The circumferential wall 128 is connected to the main body 122 of the housing 120 by a joining ledge 129 (shown in Fig. 3A). The ledge 129 provides a first abutment surface (facing in a proximal direction) which prevents the flange 16 from moving in the distal direction relative to the housing 120. The ledge 129 also provides a support surface for supporting the proximal end of the spring 24. The engagement of the spring 24 with the syringe housing 120 will be explained in more detail below with respect to Figs. 6A-6C.

The flange support 130 also comprises a plurality of clips 131 that provide a second abutment surface (facing in a distal direction), which prevents or limits the flange 16 of a syringe 1 inserted into the device 2 from moving in an axial direction with respect to the housing 120. As shown in Fig. 2 (and Fig. 3A) the flange support 130 defines a recess between the proximal facing abutment surface and the distal facing abutment surface in which a flange 16 of the syringe 1 is confined to secure the syringe within the housing 120. In the embodiment shown in Figs. 2 and 3A, the flange support 130 comprises four clips 131. However, the skilled person will appreciate that other configurations for confining a flange 16 between opposing abutment surfaces are also possible and fall within the scope of the invention.

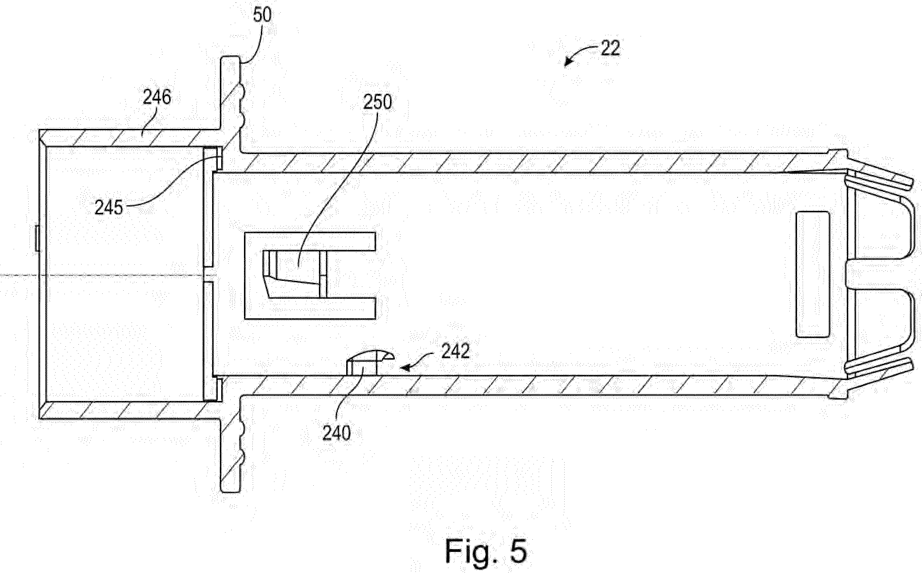

The syringe housing 120 can also comprises one or more recesses 122A in the main portion 122 of the housing 120. The recess 122A may receive a latch arm 250 (shown in Figs. 5, 6, 7A, and 7B) provided on the needle shield 22, which will be described in further detail below. One example of a device comprising a latch arm configured to engage a recess of the syringe housing is described in WO02012/ 66527.

The syringe housing 120 also comprises a track 136 (two tracks on opposing sides of the main body 122 may also be provided, or three or more tracks). The track 136 comprises a rectilinear portion 138, which extends along the main body 122 of the syringe housing 120 in a direction parallel to the longitudinal axis X-X. Since the rectilinear portion 138 of the track 138 defines the maximum axial travel of the needle shield 22 relative to the housing 120 (as described in further detail with reference to Figs.6A-6C, the first rectilinear portion 138 of the track 136 advantageously has a length longer than the length of the needle. A second track portion 140 is in communication with the first track portion and extends from a first end in communication with the proximal end of the first rectilinear portion 138 of the track 136 to a second end positioned distal of the proximal end of the first rectilinear portion of track 138. The second track portion 140 preferably connects to the first portion 138 such that the track 136 comprises an acute angle between the first portion 138 and the second portion 14. Optionally, a third rectilinear track portion 142 may extend from the second end of the second track portion 140. The third track portion 142 may extend substantially parallel to the first track portion 138. The track 136 defines a path along which a pin (not shown in Fig. 2) on the needle shield 22 may travel. The track 136 forms part of a locking mechanism for preventing relative movement between the needle shield 22 and the syringe housing 120 until after the injection has been completed. The locking mechanism will be described in more detail with reference to Fig. 6A-6C.

The syringe housing 120 may further comprise tongues 150, which extend from a distal end of the main housing portion 122. The tongues 150 are connected to the main body 122 of the housing 120 and are angled towards the longitudinal axis X-X of the housing 120 as they extend in the distal direction such that a distance separating the tongues 150 at their distal ends is less than an internal diameter of the main body 122 of the housing 120. The tongues 150 can be configured to be spaced from each other by a distance that is less than or equal to an outer diameter of, for example, a rigid needle shield provided on a syringe 1 inserted into the device. The tongues 150 are configured to flex outwardly from their initial position (shown in Fig. 2) to accommodate the needle shield when a syringe 1 is inserted into the device. When the needle shield 19 is removed from the device, the tongues return to the position shown in Fig. 2. Since the distance separating the tongues 150 is less than the diameter of the removed needle shield, the tongues 150 prevent the device from being recapped, which is a common cause of needle stick injuries and a leading cause of users mistakenly attempting to reuse a spent device.

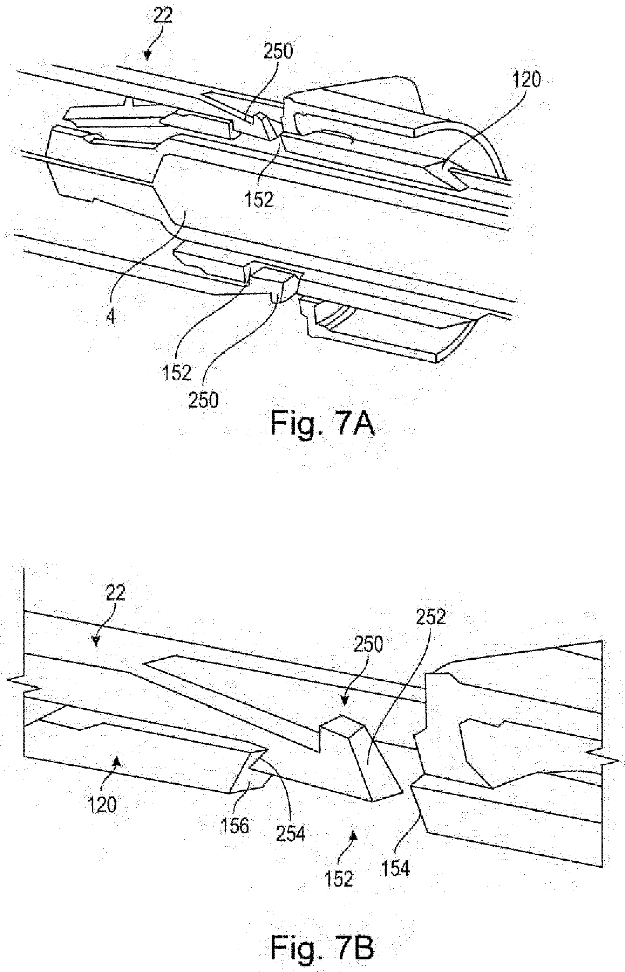

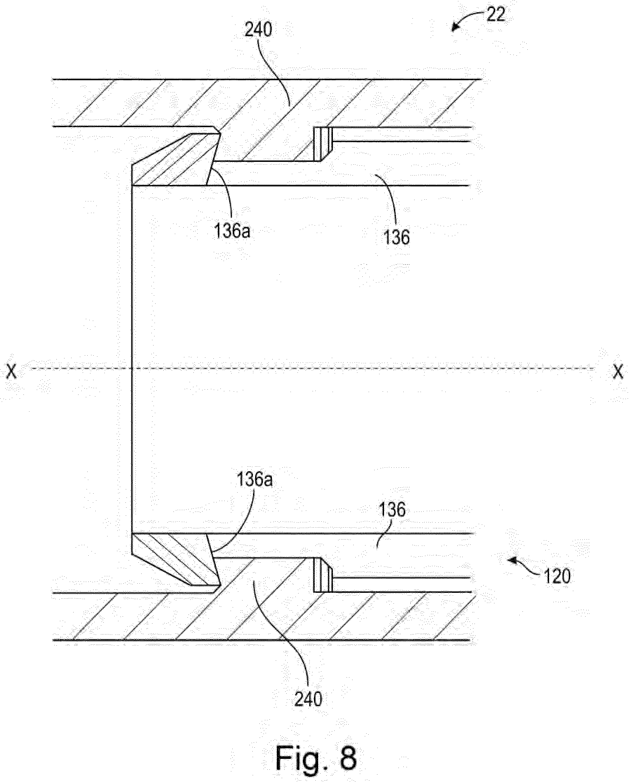

The internal components of the needle shield 22 will now be described further with reference to Fig. 5. As shown in Fig. 5, the needle shield 22 comprises a generally hollow tubular body. The hollow tubular body is configured to receive the syringe housing 120, as shown in Fig. 1. At least one pin 240 extends radially inwards from an inner surface of the needle shield 22 and is configured to engage the track 136 in the syringe housing 120 (track 136 is shown in Fig. 2 and Fig. 7). Advantageously, the pin 240 is provided with an undercut 242 on the distal side of the pin 240. As will become apparent with further reference to Figs. 6A-6C and Fig. 8, the undercut 242 allows the pin 240 to engage the distal end of the track 136. A support surface 245 for the spring 24 is provided within a circumferential spring retaining wall 246. The support surface 245 is configured to support a distal end of the spring 24. At least one latch arm 250 is provided in the main body of the needle shield 22. The latch arm 250 is configured to engage the locking recess 152 provided in the syringe housing 120 (see Fig. 2) when the latch arm 250 is brought into register with the locking recess 152 when the shield 22 is in the extended position shown in Fig. 6C. The engagement between the latch arm 250 and the locking recess 152 will be explained in further detail with reference to Figs.7A and 7B.

The interaction between the syringe housing 120 shown in Fig. 2 and the needle shield 22 shown in Fig. 5 will now be described in more detail with reference to Figs. 6A-6C. Fig. 6A shows the device 2 in an injection ready state. In this position, the spring 24 is compressed between the support surface 145 provided on the needle shield 22 and the ledge 129 of the syringe housing 120. The spring 24, in its compressed state, acts to urge the needle shield 22 in a distal direction with respect to the syringe housing 120. However, the pin 240 prevents distal movement of the needle shield 22 with respect to the syringe housing 120 because the pin 240 is confined to the track 136 and is urged towards the closed end of the second portion 142 of the track (see also Fig. 2).

Fig. 6B shows the device immediately after injection, and after a user pushes the syringe housing 120 in a distal direction, as illustrated with arrow A, further compressing the spring. As shown in Fig. 6B, as the user presses the syringe housing 120 in a distal direction with respect to the needle shield 22, the pin 240 rides along the second portion 140 of the track 136. Because the second portion of the track is angled with respect to the longitudinal axis, this causes rotation of the syringe housing 120 relative to the needle shield 22 to bring the pin 240 into alignment with the rectilinear portion 138 of the track 136 (see Fig. 6B).

Referring now to Fig. 6C, with the pin 24 in alignment with the rectilinear portion 138 of the track 136, the needle shield 22 is freely able to move in the distal direction with respect to the syringe housing 120 under the influence of the spring 24. This advance the needle shield 22 into the position shown in Fig. 6C, in which the needle of a syringe assembled into the device is covered by the shield 22.

As can also been seen from Figs. 6A to 6C, movement of the needle shield 22 from the retracted position shown in Fig. 6A to the extended position shown in Fig. 6C moves the latch arms 250 out of engagement with the recess 122A (Fig. 6A) and into engagement with locking recess 152 (Fig. 6C). Engagement of the latch arms 250 with locking recess 152 prevents the device 2 from being collapsed against the bias of the spring 24 to re-expose the needle. The engagement of the latch arms 250 with the locking recess 152 may also prevent the spring 24 from decoupling the syringe housing 120 from the needle shield 22, as will be explained in more detail with reference to Figs. 7A and 7B.

Figs. 7A shows a cross-sectional view of the distal end of the device 2 with the latch arm 250 engaged with the locking recess 152. Fig. 7B shows an enlarged view of the arrangement shown in Fig. 7A. As shown in Fig. 7A, the latch arm 250 is configured to secure (or assist in securing) the device 2 in the safe position shown in Fig. 6C after an injection has been completed. The latch arm 250 extends from a fixed end connected to the main body of the needle shield 22 to a free end. The latch arm 250 is configured to slide along the outer surface of the syringe housing 120 until it reaches the locking recess 152. The latch arm 250 is biased radially inwardly into a position in which it engages the locking recess 152 such that when the free end of the latch arm 250 is brought into register with the locking recess 152, the latch arm 250 engages the locking recess 152, as shown in Fig. 7A.

As shown in Fig. 7B, the latch arm 250 comprises first (proximal) stop surface 252 and a second (distal) stop surface 254. The first stop 252 provided at the free end of the latch arm 250 faces in a generally proximal direction and is configured to engage the proximal edge surface 154 of the locking recess 152. The second stop surface 254 formed as an undercut in the latch arm 250 faces in a generally distal direction and is configured to engage the distal edge surface 156 of the locking recess 152. The first stop surface 252 of the latch 250 and the proximal edge surface 154 of the locking recess 152 are angled with respect to the longitudinal axis X-X of the device 2 (e.g. extend in a plane that intersects the longitudinal axis X-X at a non-perpendicular angle). The edge surface 154 may be angled such that an angle defined between the sloping surface 154 and the longitudinal axis X-X is acute. The stop surface 252 may also be angled such that an angle defined between the surface 252 and the longitudinal axis is obtuse. In at least some embodiments, the angle of the proximal stop surface 252 is between 10 and 20 degrees with respect to an axis perpendicular to the longitudinal axis X-X, more preferably approximately 15 degrees. Similarly, the proximal edge surface 156 of the recess 152 may be angled with respect to the perpendicular axis by between 10 and 20 degrees, preferably approximately 15 degrees.

During an attempt to collapse the device 2 after use, the proximal stop surface 252 is brought into contact with the proximal edge surface of the locking recess 152, thereby preventing proximal movement of the needle shield 22 with respect to the syringe housing 120 due to abutment of the surfaces 252, 154. Moreover, due to the complementary inclines of the surfaces 252, 254, as force is applied against the latch arm 250 as it is brought into contact with the proximal edge of the locking recess 152, the force acts to deflect the latch arm radially inwardly, towards the longitudinal axis X-X, thereby wedging the proximal stop surface 252 under the proximal edge surface 154 and ensures that the latch arm 250 does flex radially outward to disengage the latch arm 250 from the locking recess 152.

Referring again to Fig. 7B, the distal stop surface 254 acts to resist movement of the needle shield 22 in the distal direction relative to the syringe housing 120, for example under the influence of the spring 24. As shown in Fig. 7B, if the needle shield 22 were to be urged in a distal direction relative to the syringe housing 120, e.g. under the influence of the spring or during an attempt to disassemble the needle shield 22 from the syringe housing 120, the distal stop surface 254 will be brought into contact with the distal edge surface 156 of the locking recess 152. The abutment of the distal stop surface 254 with the edge surface 156 of the recess serves to prevent (or resist) distal movement of the needle shield 22 with respect to the syringe housing 120. In at least some embodiments, the distal stop surface 254 is angled towards the longitudinal axis X-X, such that the distal stop surface 254 faces in a generally distal direction and is angled towards the longitudinal axis X-X in the distal direction to form an acute angle between the surface and the longitudinal axis. Optionally, the distal edge surface 156 of the locking recess 152 may be angled in a complementary manner, e.g. such that the surface faces in a generally proximal direction and forming an obtuse angle between the surface 156 and the longitudinal axis 156. By providing angled surfaces 254, 256 (one or both surfaces), force exerted against the distal stop surface 254 by the edge surface 156 of the locking recess 152 acts to deflect the latch arm 150 radially inwardly, further into engagement with the locking recess 152. In at least some embodiments, the angle of the distal stop surface 254 is between 10 and 20 degrees with respect to an axis perpendicular to the longitudinal axis X-X, more preferably approximately 15 degrees. Similarly, the distal edge surface 154 of the recess 152 may be angled with respect to the perpendicular axis by between 10 and 20 degrees, preferably approximately 15 degrees.

It will be appreciated that one latch arm 250 may be provided in engagement with one recess 152 to provide improvements over known systems and methods. However, a plurality of latch arms 250 configured to engage a plurality of locking recesses 152 may further reduce the likelihood of collapse or disassembly of the device after use. In the embodiments shown in the drawings, two diametrically opposed latch arms 250 are provided, configured to engage two diametrically opposed locking recesses 152. Similarly, whilst only one pin 240 is shown in the cross-sectional views of 6A-6C, in at least some exemplary embodiments, the needle shield 22 comprises first and second pins 240 configured to engage respective first and second tracks 136 on the syringe housing 120.

In some embodiments, the latch arm 250 may also be configured to provide audible or tactile feedback to the user that the pin 240 has been moved out of the retaining portion 142 of the track and is in alignment with the rectilinear portion 138 of the track 136. As shown in Fig. 6A, with the device 2 in an injection ready position, the latch arm 250 engages recess 122a in the main body 122 of the syringe housing (recess 122a is shown clearly in Fig. 2). As shown in Fig. 2, the recess 122a may further comprise a longitudinally extending rib 122b, which divides the recess into a first portion and a second portion, the first portion being closer to the track 136. With the pin 240 in the position shown in Fig. 6A, the latch arm 250 is in register with and engages a first portion of the recess 122a. However, as the needle shield 22 is rotated with respect to the housing 120 (as shown in Fig. 6B), the latch arm 250 must ride over the rib 122b. Once sufficient rotation has occurred for the latch arm 250 to ride over the rib 122b, the latch arm 250 snaps into engagement with the second portion of the recess 122a. The snap action of the latch arm 250 over the rib 122b provides audible and/or tactile feedback to the user that the needle shield 22 is in a position in which the pin 240 is in alignment with the rectilinear portion 138 of the track 136 and that the needle shield 22 will be advanced under the influence of the spring 24 to the extended position shown in Fig. 6C.

As can be seen in Fig. 2, the first portion of the recess 122a comprises a ramp 123 over which the latch arm 250 passes during assembly of the safety device 2 when the syringe housing 120 is inserted into the needle shield 22. As shown in Fig, 2, the length of the ramp 123 may be longer than a similar ramp 125 at the distal edge of the second portion of the recess 122a on the opposing side of the rib 122b. If the ramp 123 were provided with a sharp incline, it is possible that a distal stop surface of the latch arm 250 (described in more detail with reference to Figs. 7A and 7B) may bear against the ramp 123 when the safety device is in the assembled condition, thereby causing the latch arm 250 to deflect radially outwards and strain the latch arm 250 prior to use. To prevent the likelihood of this occurring, the ramp 123 is preferably of sufficient length to provide the ramp 123 with a gradual slope that is unlikely to interfere with the latch arm 250 when the needle shield 22 is in the retraced position.

As will be understood by a person skilled in the art in light of the present disclosure, the ramp 125 can be configured with a sufficiently bevelled or inclined surface to aid deflection of the latch arm 250 out of the second portion of recess 122a to allow the needle shield 22 to advance in the distal direction relative to the syringe housing 120. Therefore, the grade of the ramp 125 does not necessarily need to be the same as the grade of the ramp 123.

Referring now to Fig. 8, the engagement between the pin 240 and the track 136 will be described in more detail. As described with reference to Fig. 5 above, the needle shield 22 comprises a pin 240, configured to move along the track 136. The pin 240 can comprise an undercut 242 which forms a hook configured to engage an end wall of the track 136. Fig. 8 shows an enlarged cross-sectional view of the needle shield 22 and the syringe housing 120 when the pin 240 reaches the end of the track 136 (see Fig. 6C). Fig. 8 clearly shows the undercut of the pin 240 form a hook engagement with the end wall 136a of the track 136. The undercut on the pin 240 is formed by providing recess between the pin 240 and the wall of the needle shield 22. The recess may be formed by a distal surface of the pin which is angled with respect to the longitudinal axis X-X such that an obtuse angle is formed between the distal surface of the pin 240 and the longitudinal axis X-X. Alternatively a cut-out may be provided.. In at least some embodiments, the angle distal surface of the pin 240 is between 10 and 20 degrees with respect to an axis perpendicular to the longitudinal axis X-X, more preferably approximately 15 degrees.

As shown in Fig. 8, the hook engagement between the pin 240 and the end wall 136a of the track 136 can be further enhanced by providing a complementary angled surface on the proximally facing end wall 136a of the track 136. In the embodiment shown, the angle of the end wall 136a of the track 136 corresponds to the angle of incline of the pin undercut, e.g. such that their surfaces are approximately parallel. As can be seen in Fig. 8, the undercut provided by the angled distal surface of the pin 240 creates a hook that engages the distal end wall 136a of the track 136 such that the pin 240 not only limits relative movement of the pin 240 in the distal direction, but also prevents the pin 240 from becoming disengaged from the track 136 due to transverse movement of the pin 240 relative to the track 136. In at least some embodiments, the angle distal surface of the pin 240 is between 10 and 20 degrees with respect to an axis perpendicular to the longitudinal axis X-X, more preferably approximately 15 degrees.

As will be appreciated, although the engagement of the latch arm 250 with the locking recess 152, and the engagement of the pin 240 with the end wall 136a of the track 136 provide advantages over known systems when implemented individually, safety devices combining an undercut on the latch arm 250 (described with reference to Figs. 7A and 7B) and an undercut on the pin 240 (described with reference to Fig. 8) may provide yet further security against spontaneous or accidental disassembly of the device after use. The improvements provided may be particularly advantageous where the safety device 2 is intended for use with syringe assemblies having a small volume, for example, less than 1.0 ml (e.g. 0.5 ml volume).

Moreover, although the safety features described with reference to Figs. 7A, 7B and 8 may be provided in a safety device 2 without cantilever arms 200 (see, for example, the device described in WO2012/ 66527), the combination of the cantilever arms 200 with the latch arms 250 and/or the undercut pin 240 may provide yet further improvements over known devices, especially safety devices configured for use with a range of syringe sizes (e.g. both 1.0 ml syringes and 0.5 ml syringes).

It will also be appreciated that in addition to the devices described above, the present disclosure provides a number of exemplary methods that may provide improvements over known methods.

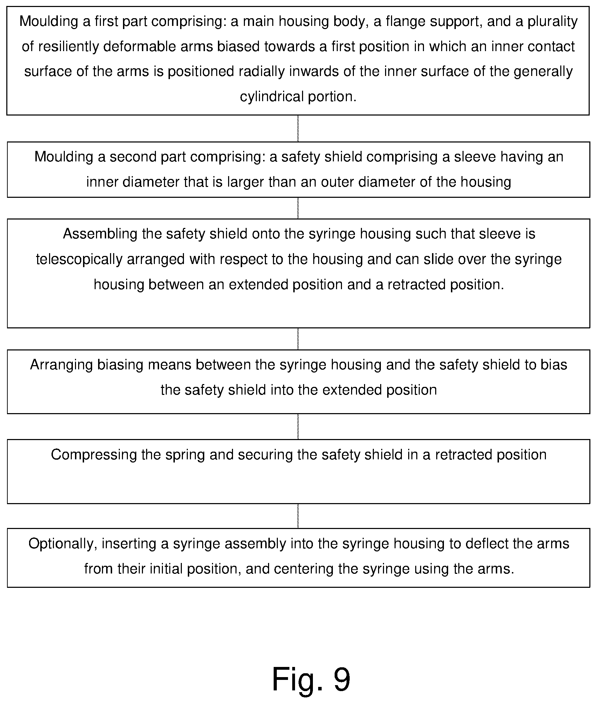

As shown schematically in Fig. 9, the disclosure also provides a method of assembling a safety device 2 and centring a syringe in the device. As shown in Fig. 9, a method of assembling a safety device may comprise the steps of: moulding a first part comprising: a main housing body, a flange support, and a plurality of resiliently deformable arms biased towards a first position in which an inner contact surface of the arms is positioned radially inwards of the inner surface of the generally cylindrical portion; moulding a second part comprising: a needle shield comprising a sleeve having an inner diameter that is larger than an outer diameter of the housing; assembling the needle shield onto the syringe housing such that the sleeve is telescopically arranged with respect to the housing and can slide over the syringe housing between an extended position and a retracted position; and compressing the spring and securing the needle shield in a retracted position. The method may optionally further comprise inserting a syringe into the syringe housing to deflect the arms from their initial position, and centring the syringe using the arms. By centring the syringe 1 within the housing, operation of the device may be improved because the syringe 1 is appropriately centred within the housing 120, the needle shield 19 may be appropriately seated between the arms 150, and the plunger 8 position can be aligned with the longitudinal axis for injection.

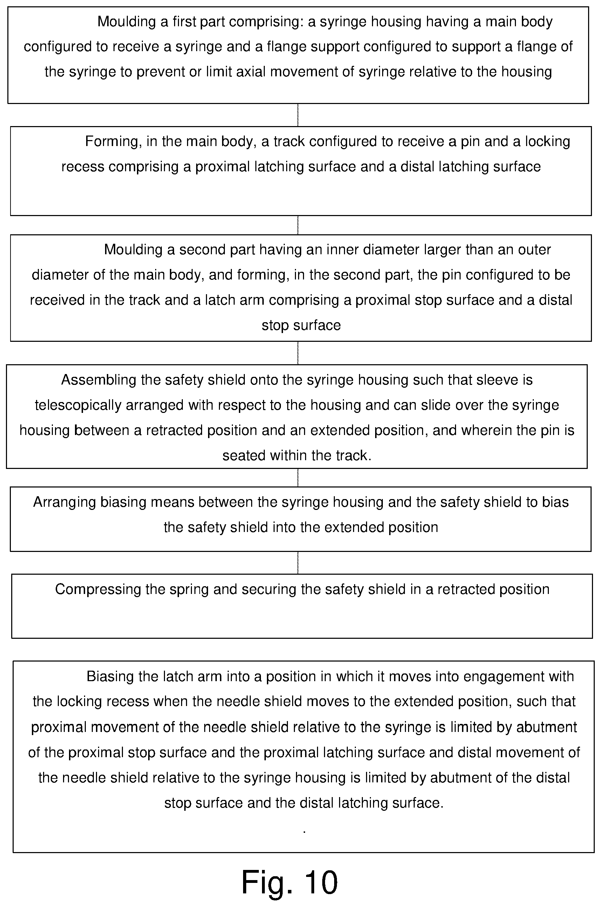

The disclosure also provides an exemplary method of manufacturing a device that prevents disassembly of the device. As shown schematically in Fig. 10, a method of manufacturing and assembling a device that prevents disassembly of the device comprises the step of: moulding a first part comprising a syringe housing 120 having a main body 122 comprising a generally cylindrical portion configured to receive a syringe 1 and a flange support 130 configured to support a flange 16 of the syringe 1 inserted into the syringe housing 120 and prevent or limit axial movement of syringe relative to the housing 120. The main body 122 is formed with a track 136 that comprises a cut-out or groove in the main body 122. The track 136 is configured to receive a pin 240 of the needle shield 22. The main body 122 further comprises a locking recess 152 comprising a proximal latching surface 154 and a distal latching surface 156. The method also comprises moulding a second part comprising a needle shield 22 comprising a sleeve having an inner diameter larger than an outer diameter of the housing 120 such that the needle shield 22 can slide over the housing. The needle shield 22 also comprises a pin 240 configured to be received in the track 136 and to move along the track 136 from a first end of the track to a second end of the track 136 as the safely shield 22 moves between the retracted and extended positions. The needle shield 22 further comprises a latch arm 250 comprising a proximal stop surface 252 and a distal stop surface 254. The method further comprises assembling the needle shield 22 with respect to the syringe housing 120 such that the needle shield 22 is telescopically arranged with respect to the syringe housing 120 and is slidable between a retracted position and an extended position. The needle shield 22 is assembled with respect to the syringe housing 120 in the retracted position. In the assembled device, the latch arm 250 is biased into a position in which it engages the locking recess 152 such that proximal movement of the needle shield 22 relative to the syringe housing 120 is limited by abutment of the proximal stop surface 252 and the proximal latching surface 154 and distal movement of the needle shield 22 relative to the syringe housing 120 is limited by abutment of the distal stop surface 254 and the distal latching surface 156 when the needle shield 22 is in the extended position.

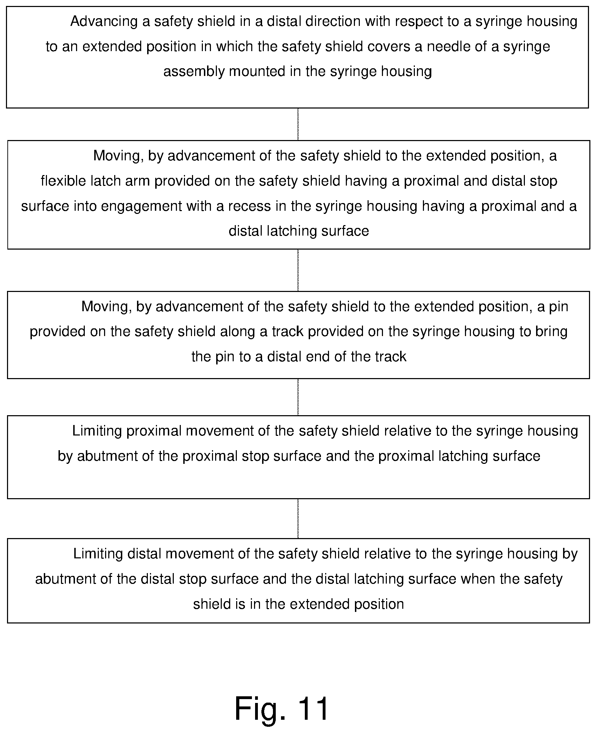

The disclosure also provides an exemplary method securing a safety device against disassembly. As shown schematically in Fig. 11, the method comprises the steps of: advancing a needle shield 22 in a distal direction with respect to a syringe housing 120 to an extended position in which the needle shield 22 covers a needle of a syringe 1 mounted in the syringe housing 120; moving, by advancement of the needle shield 22 to the extended position, a flexible latch arm 250 provided on the needle shield 22 into engagement with a locking recess 152 in the syringe housing 120; and moving, by advancement of the needle shield 22 to the extended position, a pin 240 provided on the needle shield 22 along a track 136 provided on the syringe housing 120 to bring the pin 136 to a distal end of the track 136. The method also comprises limiting proximal movement of the needle shield 22 relative to the syringe housing 120 by abutment of the proximal stop surface 252 and the proximal latching surface 154; and limiting distal movement of the needle shield 22 relative to the syringe housing 120 by abutment of the distal stop surface 254 and the distal latching surface 156 when the needle shield 22 is in the extended position.

It will be appreciated by those skilled in the art that changes can be made to the embodiments described above without departing from the invention. In particular, the methods for manufacturing set out above may further include the steps of providing any of the features described in connection with the devices described herein. In other exemplary embodiments falling within the scope of the disclosure, the respective locations of the latch arm(s) 250, pin(s) 240, locking recess(es) 152, recess(es) 122a, and track 136 may be reversed, i.e. the latch arm(s) 250 and pin(s) 240 may be located on the main body 122 of the syringe housing 120 while the locking recess(es) 152, recess(es) 122a, and track 136 may be located on the sleeve of the needle shield 22. Moreover, it will be understood that the features described in connection with one or more exemplary embodiments may be combined with features described in connection with other embodiments. Moreover, components described herein may be substituted for structurally similar or functionally equivalent components. Such modifications will be understood to fall within the scope of the present invention.

## Claims

1. A safety device (2) for a syringe (1), the safety device comprising:
a syringe housing (120) having a main body (122) comprising a generally cylindrical portion configured to receive a syringe (1), and a flange support (130) configured to support a flange (16) of the syringe (1) inserted into the syringe housing (120) and limit axial movement of the syringe (1) relative to the housing (120); and
a needle shield (22) comprising a sleeve telescopically arranged with respect to the syringe housing (120), wherein an inner diameter of the needle shield (22) is larger than an outer diameter of the housing (120) such that the needle shield (22) can slide over the housing between a retracted position in which a needle tip of the syringe (1) is exposed, and an extended position in which the needle tip of the syringe (1) is covered,
wherein the main body (122) includes a track (136) comprising a cut-out or groove in the main body (122),
wherein the needle shield (22) comprises a pin (240) configured to be received in the track (136) and to move along the track (136) from a first end of the track to a second end of the track (136) as the needle shield (22) moves between the retracted and extended positions,
wherein the main body (122) further comprises a locking recess (152) comprising a proximal latching surface (154) and a distal latching surface (156),
**characterized in that**
the needle shield (22) further comprises a latch arm (250) comprising a proximal stop surface (252) and a distal stop surface (254), and
when the needle shield (22) is in an extended position, the latch arm (250) engages the locking recess (152) such that proximal movement of the needle shield (22) relative to the syringe housing (120) is limited by abutment of the proximal stop surface (252) and the proximal latching surface (154) and distal movement of the needle shield (22) relative to the syringe housing (120) is limited by abutment of the distal stop surface (254) and the distal latching surface (156).

2. The safety device (2) of claim 1, wherein the distal stop surface (254) is angled with respect to a central longitudinal axis (X-X) of the syringe housing (120), and wherein an obtuse angle is formed between the distal stop surface (254) and the longitudinal axis (X-X).

3. The safety device (2) of any preceding claim, wherein the distal latching surface (156) is angled with respect to a central longitudinal axis (X-X) of the syringe housing (120), and wherein an acute angle is formed between the distal latching surface (156) and the longitudinal axis (X-X).

4. The safety device (2) of any preceding claim, wherein the proximal stop surface (252) is angled with respect to a longitudinal axis of the syringe housing (120), and wherein an obtuse angle is formed between the proximal stop surface (252) and a central longitudinal axis of the syringe housing (120).

5. The safety device (2) of any preceding claim, wherein the proximal latching surface (154) is angled with respect to a longitudinal axis of the syringe housing (120), and wherein an acute angle is formed between the proximal latching surface (154) and the longitudinal axis (X-X).

6. The safety device (2) of any preceding claim, wherein the pin (240) comprises an undercut between the pin (240) and a wall of the safety device (22), wherein the undercut is formed by providing a distal surface of the pin (240) that is angled with respect to the longitudinal axis (X-X), and wherein an obtuse angle is formed between the distal surface of the pin (240) and the longitudinal axis (X-X).

7. The safety device (2) of any preceding claim, wherein the track (136) comprises a distal end wall (136a), said distal end wall facing in a proximal direction, and wherein the distal end wall (136) is angled with respect to the longitudinal axis, and wherein an acute angle is formed between the distal end wall (136a) and the longitudinal axis (X-X).

8. The safety device (2) of any preceding claim, wherein the needle shield (22) comprises a plurality of latch arms (250), each configured to engage a corresponding locking recess (152) of the main body (122), and the plurality of latch arms (250) comprises two diametrically opposed latch arms (250).

9. The safety device (2) of any preceding claim, wherein each latch arm (250) is configured to engage the corresponding locking recess (152) when the pin (240) is located at the second end of the track (136).

10. The safety device (2) of any preceding claim, wherein each latch arm (250) is resiliently deformable, and wherein the housing (120) is configured to deflect the latch arm (250) as the needle shield (22) moves from the retracted to the extended position.

11. The safety device (2) of any preceding claim, further comprising a manually activated plunger.

12. The safety device (2) of any preceding claim, further comprising a biasing element (24) configured to bias the needle shield (22) into the extended position.

13. An injection apparatus comprising:
the safety device of any preceding claim; and
a syringe inserted into the housing (120).

14. The injection apparatus of claim 13, further comprising a needle cap covering a needle of the syringe, wherein an outer diameter of the needle cap is larger than or equal to an outer diameter of the syringe barrel.

15. A method of securing an injection safety device (2) against disassembly, wherein the method comprises:
advancing a needle shield (22) in a distal direction with respect to a syringe housing (120) to an extended position in which the needle shield (22) covers a needle of a syringe (1 ) mounted in the syringe housing (120);
moving, by advancement of the needle shield (22) to the extended position, a flexible latch arm (250) provided on the needle shield (22) into engagement with a locking recess (152) in the syringe housing (120), the flexible latch arm (250) comprising a proximal stop surface (252) and a distal stop surface (254), the locking recess comprising a proximal latching surface (154) and a distal latching surface (156);
moving, by advancement of the needle shield (22) to the extended position, a pin (240) provided on the needle shield (22) along a track (136) provided on the syringe housing (120) to bring the pin (240) to a distal end of the track (136);
limiting proximal movement of the needle shield (22) relative to the syringe housing (120) by abutment of the proximal stop surface (252) and the proximal latching surface (154); and
limiting distal movement of the needle shield (22) relative to the syringe housing (120) by abutment of the distal stop surface (254) and the distal latching surface (156) when the needle shield (22) is in the extended position, optionally further comprising the step of engaging the pin (240) with an end wall (136a) of the track (136).

## Patentansprüche

1. Sicherheitsvorrichtung (2) für eine Spritze (1), wobei die Sicherheitsvorrichtung umfasst:
ein Spritzengehäuse (120) mit einem Hauptkörper (122) umfassend einen allgemein zylindrischen Abschnitt, dafür gestaltet, eine Spritze (1) aufzunehmen, und einen Flanschträger (130), dafür gestaltet, einen Flansch (16) der Spritze (1), die in das Spritzengehäuse (120) eingesetzt ist, zu tragen und axiale Bewegung der Spritze (1) relativ zu dem Gehäuse (120) zu begrenzen; und
einen Nadelschutz (22) umfassend eine Hülse, die teleskopartig bezogen auf das Spritzengehäuse (120) angeordnet ist, wobei ein Innendurchmesser des Nadelschutzes (22) größer als ein Außendurchmesser des Gehäuses (120) ist, so dass der Nadelschutz (22) zwischen einer zurückgezogenen Position, in der eine Nadelspitze der Spritze (1) freiliegt, und einer ausgefahrenen Position, in der die Nadelspitze der Spritze (1) bedeckt ist, über das Gehäuse gleiten kann,
wobei der Hauptkörper (122) eine Bahn (136) aufweist, die einen Ausschnitt oder eine Nut in dem Hauptkörper (122) umfasst,
wobei der Nadelschutz (22) einen Stift (240) umfasst, dafür gestaltet, in der Bahn (136) aufgenommen zu werden und sich entlang der Bahn (136) von einem ersten Ende der Bahn zu einem zweiten Ende der Bahn (136) zu bewegen, wenn sich der Nadelschutz (22) zwischen der zurückgezogenen und der ausgefahrenen Position bewegt,
wobei der Hauptkörper (122) ferner eine Verriegelungsausnehmung (152) umfassend eine proximale Rastfläche (154) und eine distale Rastfläche (156) umfasst,
**dadurch gekennzeichnet, dass**
der Nadelschutz (22) ferner einen Verriegelungsarm (250) umfassend eine proximale Anschlagfläche (252) und eine distale Anschlagfläche (254) umfasst, und
wenn sich der Nadelschutz (22) in einer ausgefahrenen Position befindet, der Verriegelungsarm (250) in die Verriegelungsausnehmung (152) eingreift, so dass proximale Bewegung des Nadelschutzes (22) relativ zu dem Spritzengehäuse (120) durch Anliegen der proximalen Anschlagfläche (252) und der proximalen Rastfläche (154) begrenzt wird und distale Bewegung des Nadelschutzes (22) relativ zu dem Spritzengehäuse (120) durch Anliegen der distalen Anschlagfläche (254) und der distalen Rastfläche (156) begrenzt wird.

2. Sicherheitsvorrichtung (2) nach Anspruch 1, wobei die distale Anschlagfläche (254) bezogen auf eine zentrale Längsachse (X-X) des Spritzengehäuses (120) gewinkelt ist und wobei ein stumpfer Winkel zwischen der distalen Anschlagfläche (254) und der Längsachse (X-X) gebildet ist.

3. Sicherheitsvorrichtung (2) nach einem der vorstehenden Ansprüche, wobei die distale Rastfläche (156) bezogen auf eine zentrale Längsachse (X-X) des Spritzengehäuses (120) gewinkelt ist und wobei ein spitzer Winkel zwischen der distalen Rastfläche (156) und der Längsachse (X-X) gebildet ist.

4. Sicherheitsvorrichtung (2) nach einem der vorstehenden Ansprüche, wobei die proximale Anschlagfläche (252) bezogen auf eine Längsachse des Spritzengehäuses (120) gewinkelt ist und wobei ein stumpfer Winkel zwischen der proximalen Anschlagfläche (252) und einer zentralen Längsachse des Spritzengehäuses (120) gebildet ist.

5. Sicherheitsvorrichtung (2) nach einem der vorstehenden Ansprüche, wobei die proximale Rastfläche (154) bezogen auf eine Längsachse des Spritzengehäuses (120) gewinkelt ist und wobei ein spitzer Winkel zwischen der proximalen Rastfläche (154) und der Längsachse (X-X) gebildet ist.

6. Sicherheitsvorrichtung (2) nach einem der vorstehenden Ansprüche, wobei der Stift (240) eine Hinterschneidung zwischen dem Stift (240) und einer Wand der Sicherheitsvorrichtung (22) umfasst, wobei die Hinterschneidung durch Bereitstellung einer distalen Oberfläche des Stifts (240) gebildet ist, die bezogen auf die Längsachse (X-X) gewinkelt ist, und wobei ein stumpfer Winkel zwischen der distalen Oberfläche des Stifts (240) und der Längsachse (X-X) gebildet ist.

7. Sicherheitsvorrichtung (2) nach einem der vorstehenden Ansprüche, wobei die Bahn (136) eine distale Endwand (136a) umfasst, wobei die distale Endwand in eine proximale Richtung zeigt und wobei die distale Endwand (136) bezogen auf die Längsachse gewinkelt ist und wobei ein spitzer Winkel zwischen der distalen Endwand (136a) und der Längsachse (X-X) gebildet ist.

8. Sicherheitsvorrichtung (2) nach einem der vorstehenden Ansprüche, wobei der Nadelschutz (22) eine Vielzahl von Verriegelungsarme (250) umfasst, die jeweils zum Eingriff mit einer entsprechenden Verriegelungsausnehmung (152) des Hauptkörpers (122) gestaltet sind, und die Vielzahl von Verriegelungsarmen (250) zwei diametral gegenüberliegende Verriegelungsarme (250) umfasst.

9. Sicherheitsvorrichtung (2) nach einem der vorstehenden Ansprüche, wobei jeder Verriegelungsarm (250) zum Eingriff mit der entsprechende Verriegelungsausnehmung (152) gestaltet ist, wenn sich der Stift (240) an dem zweiten Ende der Bahn (136) befindet.

10. Sicherheitsvorrichtung (2) nach einem der vorstehenden Ansprüche, wobei jeder Verriegelungsarm (250) elastisch verformbar ist und wobei das Gehäuse (120) dafür gestaltet ist, den Verriegelungsarm (250) auszulenken, wenn sich der Nadelschutz (22) von der zurückgezogenen in die ausgefahrene Position bewegt.

11. Sicherheitsvorrichtung (2) nach einem der vorstehenden Ansprüche, ferner umfassend einen manuell aktivierten Stempel.

12. Sicherheitsvorrichtung (2) nach einem der vorstehenden Ansprüche, ferner umfassend ein Vorspannelement (24), dafür gestaltet, den Nadelschutz (22) in die ausgefahrene Position vorzuspannen.

13. Injektionsvorrichtung umfassend:
die Sicherheitsvorrichtung nach einem der vorstehenden Ansprüche; und
eine in das Gehäuse (120) eingesetzte Spritze.

14. Injektionsvorrichtung nach Anspruch 13, ferner umfassend eine Nadelkappe, die eine Nadel der Spritze bedeckt, wobei ein Außendurchmesser der Nadelkappe größer als ein oder gleich einem Außendurchmesser des Spritzenzylinders ist.

15. Verfahren zum Sichern einer Injektionssicherheitsvorrichtung (2) gegen Demontieren, wobei das Verfahren umfasst:
Vorschieben eines Nadelschutzes (22) in eine distale Richtung bezogen auf ein Spritzengehäuse (120) in eine ausgefahrene Position, in der der Nadelschutz (22) eine Nadel einer Spritze (1), die in dem Spritzengehäuse (120) angebracht ist, bedeckt;
durch Vorschieben des Nadelschutzes (22) in die ausgefahrene Position Bewegen eines flexiblen Verriegelungsarms (250), der an dem Nadelschutz (22) bereitgestellt ist, in Eingriff mit einer Verriegelungsausnehmung (152) in dem Spritzengehäuse (120), wobei der flexible Verriegelungsarm (250) eine proximale Anschlagfläche (252) und eine distale Anschlagfläche (254) umfasst, wobei die Verriegelungsausnehmung eine proximale Rastfläche (154) und eine distale Rastfläche (156) umfasst;
durch Vorschieben des Nadelschutzes (22) in die ausgefahrene Position Bewegen eines Stifts (240), der an dem Nadelschutz (22) bereitgestellt ist, entlang einer Bahn (136), die an dem Spritzengehäuse (120) bereitgestellt ist, um den Stift (240) an ein distales Ende der Bahn (136) zu bringen;
Begrenzen von proximaler Bewegung des Nadelschutzes (22) relativ zu dem Spritzengehäuse (120) durch Anliegen der proximalen Anschlagfläche (252) und der proximalen Rastfläche (154); und
Begrenzen von distaler Bewegung des Nadelschutzes (22) relativ zu dem Spritzengehäuse (120) durch Anliegen der distalen Anschlagfläche (254) und der distalen Rastfläche (156), wenn sich der Nadelschutz (22) in der ausgefahrenen Position befindet, gegebenenfalls ferner umfassend den Schritt des Ineingriffbringens des Stifts (240) mit einer Endwand (136a) der Bahn (136).

## Revendications

1. Dispositif de sécurité (2) pour seringue (1), le dispositif de sécurité comprenant :
un logement de seringue (120) ayant un corps principal (122) comprenant une partie généralement cylindrique configurée pour recevoir une seringue (1), et un support de bride (130) configuré pour supporter une bride (16) de la seringue (1) insérée dans le logement de seringue (120) et limiter le mouvement axial de la seringue (1) par rapport au logement (120) ; et
une protection d'aiguille (22) comprenant un manchon disposé de manière télescopique par rapport au logement de seringue (120), un diamètre intérieur de la protection d'aiguille (22) étant plus grand qu'un diamètre extérieur du logement (120) de sorte que la protection d'aiguille (22) peut coulisser sur le logement entre une position rétractée dans laquelle une pointe d'aiguille de la seringue (1) est exposée, et une position étendue dans laquelle la pointe d'aiguille de la seringue (1) est recouverte,
le corps principal (122) comprenant une piste (136) comprenant une découpe ou une rainure dans le corps principal (122),
la protection d'aiguille (22) comprenant une broche (240) configurée pour être reçue dans la piste (136) et pour se déplacer le long de la piste (136) d'une première extrémité de la piste à une seconde extrémité de la piste (136) lorsque la protection d'aiguille (22) se déplace entre les positions rétractée et étendue,
le corps principal (122) comprenant en outre un évidement de verrouillage (152) comprenant une surface de verrouillage proximale (154) et une surface de verrouillage distale (156),
**caractérisé en ce que**
la protection d'aiguille (22) comprend en outre un bras de verrouillage (250) comprenant une surface d'arrêt proximale (252) et une surface d'arrêt distale (254), et lorsque la protection d'aiguille (22) est en position étendue, le bras de verrouillage (250) vient en prise avec l'évidement de verrouillage (152) de sorte que le mouvement proximal de la protection d'aiguille (22) par rapport au logement de seringue (120) est limité par la butée de la surface d'arrêt proximale (252) et de la surface de verrouillage proximale (154) et le mouvement distal de la protection d'aiguille (22) par rapport au logement de seringue (120) est limité par la butée de la surface d'arrêt distale (254) et de la surface de verrouillage distale (156).

2. Dispositif de sécurité (2) selon la revendication 1, la surface d'arrêt distale (254) étant inclinée par rapport à un axe longitudinal central (X-X) du logement de seringue (120), et un angle obtus étant formé entre la surface d'arrêt distale (254) et l'axe longitudinal (X-X).

3. Dispositif de sécurité (2) selon l'une quelconque des revendications précédentes, la surface de verrouillage distale (156) étant inclinée par rapport à un axe longitudinal central (X-X) du logement de seringue (120), et un angle aigu étant formé entre la surface de verrouillage distale (156) et l'axe longitudinal (X-X).

4. Dispositif de sécurité (2) selon l'une quelconque des revendications précédentes, la surface d'arrêt proximale (252) étant inclinée par rapport à un axe longitudinal du logement de seringue (120), et un angle obtus étant formé entre la surface d'arrêt proximale (252) et un axe longitudinal central du logement de seringue (120).

5. Dispositif de sécurité (2) selon l'une quelconque des revendications précédentes, la surface de verrouillage proximale (154) étant inclinée par rapport à un axe longitudinal du logement de seringue (120), et un angle aigu étant formé entre la surface de verrouillage proximale (154) et l'axe longitudinal (X-X) .

6. Dispositif de sécurité (2) selon l'une quelconque des revendications précédentes, la broche (240) comprenant un dégagement entre la broche (240) et une paroi du dispositif de sécurité (22), le dégagement étant formé en fournissant une surface distale de la broche (240) qui est inclinée par rapport à l'axe longitudinal (X-X), et un angle obtus étant formé entre la surface distale de la broche (240) et l'axe longitudinal (X-X).

7. Dispositif de sécurité (2) selon l'une quelconque des revendications précédentes, la piste (136) comprenant une paroi d'extrémité distale (136a), ladite paroi d'extrémité distale faisant face dans une direction proximale, et la paroi d'extrémité distale (136) étant inclinée par rapport à l'axe longitudinal, et un angle aigu étant formé entre la paroi d'extrémité distale (136a) et l'axe longitudinal (X-X).

8. Dispositif de sécurité (2) selon l'une quelconque des revendications précédentes, la protection d'aiguille (22) comprenant une pluralité de bras de verrouillage (250), chacun configuré pour venir en prise avec un évidement de verrouillage correspondant (152) du corps principal (122), et la pluralité de bras de verrouillage (250) comprenant deux bras de verrouillage diamétralement opposés (250).

9. Dispositif de sécurité (2) selon l'une quelconque des revendications précédentes, chaque bras de verrouillage (250) étant configuré pour venir en prise avec l'évidement de verrouillage correspondant (152) lorsque la broche (240) est située à la seconde extrémité de la piste (136).

10. Dispositif de sécurité (2) selon l'une quelconque des revendications précédentes, chaque bras de verrouillage (250) étant élastiquement déformable, et le logement (120) étant configuré pour dévier le bras de verrouillage (250) lorsque la protection d'aiguille (22) se déplace de la position rétractée à la position étendue.

11. Dispositif de sécurité (2) selon l'une quelconque des revendications précédentes, comprenant en outre un piston actionné manuellement.

12. Dispositif de sécurité (2) selon l'une quelconque des revendications précédentes, comprenant en outre un élément de sollicitation (24) configuré pour solliciter la protection d'aiguille (22) dans la position étendue.

13. Appareil d'injection comprenant :
le dispositif de sécurité selon l'une quelconque des revendications précédentes ; et
une seringue insérée dans le logement (120).

14. Appareil d'injection selon la revendication 13, comprenant en outre un capuchon d'aiguille recouvrant une aiguille de la seringue, un diamètre extérieur du capuchon d'aiguille étant supérieur ou égal à un diamètre extérieur du cylindre de seringue.

15. Procédé de fixation d'un dispositif de sécurité d'injection (2) contre le démontage, le procédé comprenant :
l'avancement d'une protection d'aiguille (22) dans une direction distale par rapport à un logement de seringue (120) jusqu'à une position étendue dans laquelle la protection d'aiguille (22) recouvre une aiguille d'une seringue (1) montée dans le logement de seringue (120) ; le déplacement, par avancement de la protection d'aiguille (22) vers la position étendue, d'un bras de verrouillage flexible (250) prévu sur la protection d'aiguille (22) en prise avec un évidement de verrouillage (152) dans le logement de seringue (120), le bras de verrouillage flexible (250) comprenant une surface d'arrêt proximale (252) et une surface d'arrêt distale (254), l'évidement de verrouillage comprenant une surface de verrouillage proximale (154) et une surface de verrouillage distale (156) ;
le déplacement, par avancement de la protection d'aiguille (22) vers la position étendue, d'une broche (240) placée sur la protection d'aiguille (22) le long d'une piste (136) placée sur le logement de seringue (120) pour amener la broche (240) à une extrémité distale de la piste (136) ;
la limitation du mouvement proximal de la protection d'aiguille (22) par rapport au logement de seringue (120) par butée de la surface d'arrêt proximale (252) et de la surface de verrouillage proximale (154) ; et
la limitation du mouvement distal de la protection d'aiguille (22) par rapport au logement de seringue (120) par butée de la surface d'arrêt distale (254) et de la surface de verrouillage distale (156) lorsque la protection d'aiguille (22) est en position étendue, comprenant éventuellement en outre l'étape de mise en prise de la broche (240) avec une paroi d'extrémité (136a) de la piste (136).
